Europäisches Patentamt

European Patent Office

Office européen des brevets

(19)

(11) Publication number: **0 082 587**
**B1**

## EUROPEAN PATENT SPECIFICATION

(12)

(45) Date of publication of patent specification: **22.01.86**

(51) Int. Cl.⁴: **G 01 N 33/96, A 61 K 35/16**

(21) Application number: **82305441.6**

(22) Date of filing: **13.10.82**

(54) Preparation of storage-stable serum.

(30) Priority: **30.11.81 US 325791**

(43) Date of publication of application:
**29.06.83 Bulletin 83/26**

(45) Publication of the grant of the patent:
**22.01.86 Bulletin 86/04**

(84) Designated Contracting States:
**DE FR GB IT**

(56) References cited:
**EP-A-0 019 939**
**DE-A-2 353 035**
**DE-A-2 442 680**
**DE-A-2 927 841**
**US-A-3 955 925**
**US-A-4 007 008**

(73) Proprietor: **TECHNICON INSTRUMENTS CORPORATION**
**511 Benedict Avenue**
**Tarrytown, New York 10591 (US)**

(72) Inventor: **Engler, Phillip V.**
**21 Maple Street**
**Tarrytown New York 10591 (US)**
Inventor: **Buhl, Steven N.**
**4 South Delaware Drive**
**Nyack New York 10960 (US)**

(74) Representative: **Wain, Christopher Paul et al**
**A.A. THORNTON & CO. Northumberland House**
**303-306 High Holborn**
**London WC1V 7LE (GB)**

Courier Press, Leamington Spa, England.

## Description

This invention relates to a process for preparing a stable consistent serum especially, but not exclusively, useful for control and calibration purposes in diagnostic analyses.

Serum to be employed for calibration or control purposes in diagnostic analyses must be storable, but this has been difficult to achieve for any reasonable length of time because it tends to become turbid and is difficult to filter after standing even for short periods. For example, unprocessed or untreated liquid bovine serum when stored under refrigeration, begins to deteriorate within 48 hours. Concurrent with this deterioration, the serum sample becomes turbid which adversely affects absorbance properties and severely limits its utility. Filtration becomes a difficult time-consuming process, and even when it is achieved, the resulting serum suffers a clarity problem which reduces its utility.

European specification no. 19939 describes a process for preparing substantially completely delipified animal serum of high optical clarity for the manufacture of serum based control material. The process comprises removing all the electrolytes by mixing the serum with a mixed anion-cation ion exchange resin which reduces the pH to $5.2 \pm 0.3$, and separating the supernatant. U.K. specification 1486787 describes a process for stabilising body liquids such as serum in which process part of the lipoproteins and also other unstable serum components are removed. The process involves treating the serum with a salt of an acridine or quinoline compound, and removing the precipitate and any free remaining acridine or quinoline compound.

U.S. specifications nos. 3932943 and 3928566 describe a process for producing lyophilised serum (without removal of macromolecular lipids or lipid proteins) and upon subsequent dissolution the lyophilised product is said to form a solution of greater clarity than prior art products. However, the process involves a quite complex freezing and lyophilisation procedure.

In both these known processes, lipids and/or lipoproteins are removed from the sera and, strictly, these substances must be replaced in the sera before it can be used as a true clinical control.

We have now devised a process for treating serum which does not involve the removal of macromolecular lipids or lipoproteins, and by which a stable consistent serum having clear optical properties and improved filterability can be obtained. The process of the present invention does not involve complex freezing procedures.

According to the invention, a component or components of the serum are insolubilised by reducing the pH of the serum by an acid addition treatment, the insolubilised component or components are separated from the serum, and the separated serum is then stabilised by raising its pH.

The invention also includes a process of preparing a serum intermediate from serum without significant loss of macromolecular lipid components, which process comprises the steps of insolubilising a component or components of said serum by rendering the pH of said serum more acidic by an acid addition treatment, and separating said insolubilised component or components from said serum. The resulting serum intermediate can be stabilised as described above, or it may be lyophilised and, when required, reconstituted and stabilised.

The novel processed sera of this invention, because of the highly desirable properties arising from such processing, are rendered well suited for extended storage. As a result of improved lyophilisation and rehydration properties, they are especially useful for application as calibrators and controls.

Serum processed according to this invention can be stored in the frozen state at $-20°C$ for longer than 8 months without significant deterioration and without becoming turbid as compared to unprocessed bovine serum which deteriorates and becomes turbid within 6 months under similar conditions. Even more striking is the comparison at refrigerated liquid storage, i.e. at $2-8°C$. A processed bovine serum according to this invention will be storage stable under these conditions for up to about 2 weeks, whereas an unprocessed sample deteriorates within 48 hours. This latter storage stability property is especially significant since it allows a technician to prepare and store relatively large quantities of serum formulation for use over a period of 10 days or more without risk of having to discard it because of deterioration. Obviously, it is also a time saving factor to be able to prepare larger quantities rather than small amounts in repeated fashion.

The process of the invention provides a stable, very consistent serum having clear optical properties without significant loss of macromolecular lipid components. The resulting processed serum exhibits physical properties which render it especially well suited to additional processing insofar as it has improved filterability and thermodynamic properties. These properties make the serum well suited for extended storage as a sterile liquid or in the frozen state. The processed serum also exhibits improved lyophilisation and rehydration properties for use in making calibrators and controls.

In the process of this invention for preparing such processed serum, the raw serum, e.g. bovine serum, is subjected to an insolubilisation step wherein the pH is reduced by the addition of an organic or inorganic acid. Preferably, the pH is adjusted below about 5, and more preferably to a pH range of 4 to 5, most preferably to a pH range from 4.4 to 4.6.

Typically, for employment as a calibrator or control, bovine serum is used, but other mammalian sera can be processed by this method including equine and human serum. Additionally, plasma or defibrinated plasma can be used in this process.

The insolubilisation step is generally effected by pH adjustment employing any organic or inorganic acid which does not interfere with the inherent nature of the serum or its prospective application. Typical acid examples are hydrochloric acid and acetic acid.

The cloudy serum after the insolubilisation step is then treated by, for example, filtration or centrifugation, to provide a clear liquid. The reason for the turbidity which results from pH lowering is not clearly understood. While not wishing to be held to any particular mechanism without further investigation, it is theorised that the acid precipitable molecules and/or fractions are protein or protein-related. In any event, the removal of this material appears to provide a serum that is inherently more stable and upon freezing has a better "crystal structure". The removal of the "precipitate" by filtration can be effected by any suitable filtration procedure. For example, the adjusted pH serum can be filtered through a filter medium of filter paper such as an asbestos-cellulose, cellulose or a fiberglass filter medium. It has been found suitable to include a filter aid such as diatomaceous earth.

After filtration (or separation by other means), the serum is stabilised or neutralised, by raising the pH, preferably to 7 to 8, more preferably 7.5, by the inclusion of any organic or inorganic base which does not interfere with the inherent nature of the serum or its prospective use. Representative examples include sodium hydroxide and trihydroxyaminomethane.

The resulting processed serum has excellent storage properties, as discussed above, for it to be stored in substantial quantities for eventual use in or as calibration or control materials in diagnostic analyses. The processed serum can be stored as such, or it can be lyophilised, optionally in either case after the incorporation of conventionally employed analytes. The lyophilised material is extremely stable and can be easily reconstituted. Typical analytes include glucose, potassium, alkaline phosphatase, creatine kinase, urea, lactate dehydrogenase (LDH), alanine-amino transferase, triglycerides and cholesterol.

This invention also includes a serum intermediate obtained by insolubilising a component or components of the serum by rendering the pH of said serum more acidic and separating said insolubilised component or components from said serum. The resulting serum can be lyophilised or can thereafter be stabilised as described above. The lyophilised material can be reconstituted and then stabilised. In general, the analytes are not incorporated until the intermediate serum is stabilised.

In order that the invention may be more fully understood, the following Examples are given by way of illustration only.

Example I

A pool of bovine serum (1000 l, pH 7.5) is adjusted to a pH of 4.4—4.6 with hydrochloric acid (3 M). At this pH, the serum becomes cloudy. A filter aid, e.g. diatomaceous earth, (0.5% by weight) is added to this cloudy serum which is then passed through a filtration system, a filter housing containing CUNO 50S filter pads. The filtrate is adjusted to a pH of 7.5—7.7 with sodium hydroxide (4 M). The resulting processed serum exhibits the properties described herein.

Continuous flow centrifugation at 720,000 g with a 10 min dwell time can be substituted for the filtration step.

Example II

The processed serum of Example I is made into a calibrator or control by adding predetermined amounts of analytes glucose, potassium, alkaline phosphatase, creatine kinase, urea, lactate dehydrogenase (LDH), alanine-amino transferase (SGPT), triglycerides, cholesterol and other analytes used to calibrate any assay method or monitor control of any assay, especially those of clinical importance. A more specific example of a control material is described in the Table which follows:

## Table

| Analyte | Normal Value* |
|---|---|
| Albumin | 3.9 g/dl |
| Alk. Phos. | 100 U/L |
| Calcium | 7.0 mg/dl |
| CPK | 200 U/L |
| Chloride | 95 meq/L |
| Cholesterol | 220 mg/dl |
| $CO_2$ | 32 meq/L |
| Creatinine | 1.5 mg/dl |
| Glucose | 60 mg/dl |
| Inorganic Phosphorus | 2.5 mg/dl |
| LDH | 225 U/L |
| Potassium | 3.5 meq/L |
| Sodium | 140 meq/L |
| Total Bilirubin | 1.0 mg/dl |
| Total Protein | 7.0 g/dl |
| SGOT (aspartate amino-transferase) | 40 U/L |
| SGPT | 40 U/L |
| BUN (blood urea nitrogen) | 20 mg/dl |
| Triglycerides | 120 mg/dl |
| Uric Acid | 4.0 mg/dl |
| Total Iron | 150 ug/dl |
| GGT (gamma glutamyl transferase) | 40 U/L |
| Direct Bili. (bilirubin) | 0.1 mg/dl |

* Normal values as defined by assays on Technicon's SMAC, a registered Technicon trademark.

The formulated serum is then dispersed into aliquots, lyophilised or preserved for storage before use.

Prior to addition of analytes, the sodium and chloride levels of the processed serum can be reduced by any convenient procedure such as dialysis or diafiltration, and the serum concentrated.

The serum can also be concentrated and the sodium and chloride levels reduced before pH treatment and filtration or centrifugation.

### Example III

The procedure of Example I is repeated, and after filtration, the serum is lyophilised. This lyophilised material can be reconstituted and stabilised as described in Example I.

### Example IV

The procedure of Example I is essentially repeated except that human serum is used in place of bovine serum.

**Claims**

1. A process for preparing a stable, consistent serum having clear optical qualities and improved filterability without significant loss of macromolecular lipid components which comprises the steps of insolubilising a component or components of said serum by reducing the pH of the serum by an acid addition treatment, separating said insolubilised component or components from the serum, and stabilising the separated serum by raising its pH.

2. A process according to claim 1, wherein the serum is bovine serum or plasma, or human serum or plasma.

3. A process according to claim 1 or 2, wherein said insolubilisation step is effected by reducing the pH of the serum to below about 5.

4. A process according to claim 3, wherein the pH is reduced by the addition of an organic or inorganic acid, for example hydrochloric acid or acetic acid, which does not interfere with the inherent nature of the serum or its prospective use.

5. A process according to any of claims 1 to 4, wherein said separation step is effected by filtration through a filter medium, preferably employing a filter aid such as diatomaceous earth, or by centrifugation.

6. A process according to any of claims 1 to 5, wherein said stabilising step is effected by raising the pH of the separated serum to the range 7 to 8.

7. A process according to claim 6, wherein the pH is raised by the addition of an organic or inorganic base preferably sodium hydroxide or trihydroxyaminomethane, which does not interfere with the inherent nature of the serum or its prospective use.

8. A process according to any of claims 1 to 7, wherein the separated stabilised serum is frozen or lyophilised.

9. A process of preparing a serum intermediate from serum without significant loss of macromolecular lipid components, which comprises the steps of insolubilising a component or components of said serum by rendering the pH of said serum more acidic by an acid addition treatment, and separating said insolubilised component or components from said serum, and if desired freezing or lyophilising the resulting serum.

10. The use of sera treated by the method of any preceding claim, in diagnostic analyses.

## Revendications

1. Procédé pour la préparation d'un sérum stable, uniforme doté de limpidité optique et d'une filtrabilité améliorée sans perte notable de constituants lipides macromoléculaires, qui comprend les opérations d'insolubilisation d'un ou des constituants dudit sérum en réduisant le pH du sérum par traitement d'addition d'acide, de séparation du sérum ledit ou lesdits constituants insolubilisés et de stabilisation du sérum séparé en élevant son pH.

2. Procédé selon la revendication 1, où le sérum est du sérum ou plasma bovin ou du sérum ou plasma humain.

3. Procédé selon la revendication 1 ou 2, où l'on effectue ladite opération d'insolubilisation en réduisant le pH du sérum au-dessous d'environ 5.

4. Procédé selon la revendication 3, où l'on réduit le pH par addition d'un acide organique ou inorganique, par exemple acide chlorhydrique ou acide acétique, qui ne trouble pas la nature propre du sérum ou à son usage prévu.

5. Procédé selon l'une quelconque des revendications 1 à 4, où l'on effectue ladite opération de séparation par filtration à travers un milieu filtrant, en utilisant de préférence un adjuvant de filtration tel que terre d'infusoires, ou par centrifugation.

6. Procédé selon l'une quelconque des revendications 1 à 5, où l'on effectue ladite opération de stabilisation en portant le pH du sérum séparé dans la gamme de 7 à 8.

7. Procédé selon la revendication 6, où l'on élève le pH par addition dune base organique ou inorganique, de préférence hydroxyde de sodium ou trihydroxyaminométhane, qui ne trouble pas la nature propre du sérum ni à son usage prévu.

8. Procédé selon l'une quelconque des revendications 1 à 7, caractérisé en ce qu'on congèle ou lyophilise le sérum séparé stabilisé.

9. Procédé de préparation de produit intermédiaire de sérum à partir de sérum sans perte notable de constituants lipides macromoléculaires, qui comprend les opérations d'insolubilisation d'un constituant ou de constituants dudit sérum en rendant le pH du sérum plus acide par traitement d'addition d'acide et de séparation du sérum du ou des constituants insolubilisés, et de congélation ou lyophilisation du sérum résultant si on le désire.

10. Utilisation de sérums traités par le procédé selon l'une quelconque des revendications précédentes en analyse diagnostique.

## Patentansprüche

1. Verfahren zur Herstellung eines chemisch und physikalisch stabilen, optisch klaren und leicht filtrierbaren Serums ohne beträchtlichen Verlust makromolekularer Lipidkomponenten, dadurch gekennzeichnet, daß man eine Komponente oder Komponenten des Serums durch Erniedrigen des pH-Wertes des Serums durch Säurezusatz unlöslich macht, die unlöslich gemachte Komponente oder die unlöslich gemachten Komponenten von dem Serum abtrennt und das von dieser Komponente oder diesen Komponenten befreite Serum durch Erhöhen seines pH-Wertes stabilisiert.

2. Verfahren gemäß Anspruch 1, dadurch gekennzeichnet, daß das Serum Rinder- oder Humanserum oder -plasma ist.

3. Verfahren gemäß Anspruch 1 oder 2, dadurch gekennzeichnet, daß das Unlöslichmachen durch Erniedrigen des pH-Wertes des Serums auf unter etwa 5 bewirkt wird.

4. Verfahren gemäß Anspruch 3, dadurch gekennzeichnet, daß der pH-Wert durch Zusetzen einer organischen oder anorganischen Säure, welche die dem Serum innewohnenden Eigenschaften oder seine in Aussicht genommene Verwendung nicht beeinträchtigt, beispielsweise Salzsäure oder Essigsäure, erniedrigt wird.

5. Verfahren gemäß einem der Ansprüche 1 bis 4, dadurch gekennzeichnet, daß die Abtrennung durch Filtrieren durch ein Filtermedium bewirkt wird, wobei vorzugsweise eine Filterhilfe wie Diatomeenerde verwendet wird, oder durch Zentrifugieren bewirkt wird.

6. Verfahren gemäß einem der Ansprüche 1 bis 5, dadurch gekennzeichnet, daß das Stabilisieren durch Erhöhen des pH-Wertes des abgetrennten Serums auf einen Wert von 7 bis 8 bewirkt wird.

7. Verfahren gemäß Anspruch 6, dadurch gekennzeichnet, daß der pH-Wert durch Zusatz einer organischen oder anorganischen Base, welche die dem Serum innewohnenden Eigenschaften oder seine vorgesehene Verwendung nicht beeinträchtigt, vorzugsweise von Natriumhydroxid oder Trihydroxyaminomethan, erhöht wird.

8. Verfahren gemäß einem der Ansprüche 1 bis 7, dadurch gekennzeichnet, daß das abgetrennte, stabilisierte Serum gefroren oder lyophilisiert wird.

9. Verfahren zur Herstellung eines Serumzwischenproduktes aus Serum ohne beträchtlichen Verlust an makromolekularen Lipidkomponenten, dadurch gekennzeichnet, daß man eine Komponente oder Komponenten des Serums unlöslich macht, indem man den pH-Wert des Serums durch Säurezusatz erniedrigt und die unlöslich gemachte Komponente oder die unlöslich gemachten Komponenten von dem Serum abtrennt und gewünschtenfalls das erhaltene Serum gefriert oder lyophilisiert.

10. Verwendung von Seren, die nach dem Verfahren gemäß einem der vorhergehenden Ansprüche behandelt worden sind, für diagnostische Analysen.